# EUROPEAN PATENT APPLICATION

(11) **EP 0 743 363 A2**
(43) Date of publication of application: **20.11.1996**
(21) Application number: 96400902.1
(22) Date of filing: 26.04.1996
(51) Int. Cl.: C12N 9/24, C12P 19/14, C12N 1/12, C12N 1/20

(54) **Novel beta-agrase and microorganism generating the same**

(30) Priority: 27.04.1995 JP 125610/95
(71) Applicant: KABUSHIKI KAISHA YAKULT HONSHA, Minato-ku Tokyo 105 (JP)
(72) Inventor: Araki, Toshiyoshi, Tsu-shi, Mie-ken (JP)
(74) Representative: Warcoin, Jacques

(57) **Abstract**

Novel two types of β -agarases derived from a marine bacterium screened and isolated from marine sediment, include β -agarase A of a molecular weight of about 90,000 and β -agarase B of a molecular weight of about 98,000. β -agarase A has an enzyme activity of hydrolyzing agarose and porphyran mainly into neoagarotetraose and neoagaro hexaose. β -agarase B has an enzyme activity of hydrolyzing agarose and porphyran mainly into neoagarobiose. These novel two types of β -agarases are used for preparing an oligosaccharide and for preparing the protoplast of seaweed cells.

## Description

### BACKGROUND OF THE INVENTION

### 1. Field of the Invention

The present invention relates to a novel β -agarase, namely a novel enzyme having the β -agarase activity, and a novel microorganism generating the enzyme. Furthermore, the present invention relates to a method for preparing the enzyme, and a method for preparing oligosaccharides and protoplasts using the enzyme.

### 2. Background Art

Agar and porphyran comprise seaweed-specific polysaccharides, which are contained, in a certain species of red algae (seaweed), as the cell wall matrix thereof. It is currently known that the oligosaccharides in agar and porphyran have pharmacological actions such as hypotensive action, anti-tumor action and an action of activating enterokinesis, and a bacteriostatic action of certain species of microorganisms, in addition to the utility as an investigative reagent. The oligosaccharide of porphyran in particular has sulfate groups absorbing and excreting the sodium ion outside bodies. Therefore, the oligosaccharide is effective for preventing the excess intake of salts.

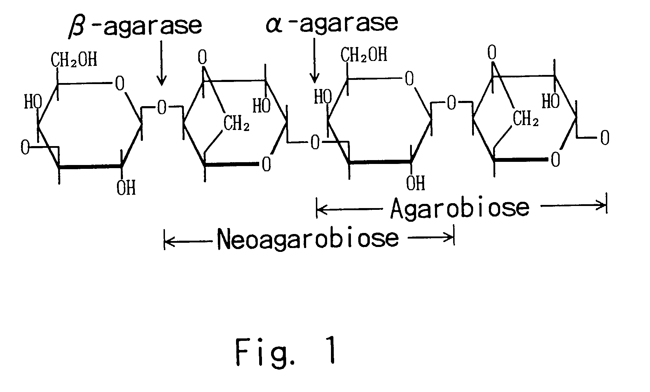

Agar is composed of two chemical components, namely agarose contained in the linear chain galactan, and agaropectin. Fig.l shows the structure of the units composing agarose. As shown in Fig.1, agarose is one of neutral polysaccharides of gelling properties, comprising an alternate linear linking of agarobiose, namely 4-o-β -D-galactopyranosyl-3,6-anhydro-L-galactose and neoagarobiose, namely 3-0-(3,6-anhydro-α -L-galactopyranosyl)-D-galactose.

On contrast, the chemical structure of agaropectin is so complex that it has not yet been elucidated completely. However, it is evident currently that agaropectin comprises D-galactose and 3,6-anhydro-L-galactose as the principal components and other lesser amounts of sulfates, glucuronic acid, pyruvic acid and the like. The pyruvic acid is in the ketal bonding with the C-4 and C-6 of the D-galactose residue in the agarobiose unit. This indicates that agarobiose is a significant structural component of agaropectin.

Alternatively, porphyran is a polysaccharide from hot-water extraction of red seaweed of Porphyra spp.. The polysaccharide is a linear sulfated galactan, including principal components of 3-β -D-galactosyl and 4-α -L-galactosyl units. Porphyran is produced as a gelling agent at a small scale in Japan and China.

For the purpose of the effective application of seaweed as a marine biological resource and for the purpose of developing a method for preparing an agaro-oligosaccharide as a physiologically active substance, investigations have been made of a novel agarose hydrolase and a novel microorganism having the agarose generation potency (Japanese Patent Laid-open No. Hei 1-228465). As to the activity of hydrolyzing agarose into neoagarose oligosaccharide, in particular, almost no microorganism is currently known, having the hydrolyzing potency of neoagarotetraose (tetrasaccharide).

### SUMMARY OF THE INVENTION

The present inventors have made investigations in such circumstances, and have found that a marine bacterium isolated from marine sediment generates an enzyme efficiently hydrolyzing agarose and porphyran as seaweed polysaccharides and also efficiently hydrolyzing neoagarotetraose, which has been hydrolyzed with much difficulty via conventional agarases. Further, the inventors have isolated and purified two types of β -agarases from the bacterium. Thus, the present invention has been achieved.

It is an objective of the present invention to provide a novel β -agarase having excellent activities, in particular the activity of hydrolyzing neoagarotetraose and porphyran.

The said β -agarase in accordance with the present invention includes two types of agarases, mainly a first β -agarase and a second β -agarase as described below.

The first β -agarase is an enzyme having a catalytic activity for the hydrolysis of agarose into lower molecular substances, mainly neoagarotetraose (tetrasaccharide) or neoagarohexaose (hexasaccharide). The specific physico-chemical properties will be described hereinafter.

The second β -agarase is an enzyme having a catalytic activity for the hydrolysis of agarose into lower molecular substances, mainly neoagarobiose (disaccharide). The specific physico-chemical properties will be described also hereinafter.

These first and second β -agarases are generated by culturing a microorganism having the potency of generating β -agarase, more specifically a novel microorganism Vibrio sp. PO-303, using at least one of agar, agarose or porphyran as the carbon source.

The novel microorganism Vibrio sp. PO-303 has the potency of generating the two types of enzymes, namely the first and second β -agarases, and said microorganism has been already deposited on April 21, 1995, in the Agency of Industrial Science and Technology, the Fermentation Research Institute at Ibaraki-ken, Japan, as FERM BP-5411.

According to the present invention, furthermore, it is provided a method for preparing the first or second β -agarase. The method comprises culturing a microorganism having the potency of generating β -agarase using at least one of carbon source selected from a group consisting of agar, agarose and porphyran, isolating the first or second β -agarase from the culture supernatant, and purifying the isolated first or second β -agarase.

Specifically, the microorganism having the potency of generating said β -agarase is the Vibrio sp. PO-303 (FERM BP-5411) as a novel bacterial strain.

According to the present invention, further, it is provided a method for preparing oligosaccharides using the first or second β -agarase. The method comprises a step of reacting the first or second β -agarase with at least one substrate of agar, agarose or porphyran. From the first β -agarase, mainly, neoagarotetraose (tetrasaccharide) and neoagarohexaose (hexasaccharide) are produced; from the second β -agarose, mainly, neoagarobiose (disaccharide) is produced.

According to the present invention, still furthermore, it is provided a method for preparing the protoplast of seaweed cells using the first or second β -agarase. The method comprises a step of stirring seaweed cells together with the first or second β -agarase in a buffer solution.

The first β -agarase of the present invention is an enzyme having the catalytic activity for the hydrolysis of agarose into lower molecular substances, mainly neoagarotetraose (tetrasaccharide) and neoagarohexaose (hexasaccharide), and it is identified that the enzyme has the following physico-chemical properties (a) to (g).

(a) Molecular weight:
   The molecular weight was determined by SDS-polyacrylamide gel electrophoresis (SDS-PAGE), with the result that the molecular weight is about 90,000. No report has been issued yet of any known agarase with such molecular weight.
(b) Isoelectric point:
   The result of the isoelectric focusing indicates that the enzyme has an isoelectric point of about 6.6. No report has been issued yet of any known agarase with such isoelectric point.
(c) Solubility:
   The enzyme is water soluble.
(d) Action:
   The enzyme catalyses the hydrolysis of at least agar, agarose and porphyran into low molecular substances, mainly tetrasaccharides and hexasaccharides. The enzyme does not hydrolyze κ -carrageenan or λ -carrageenan.
(e) Optimum temperature:
   45 °C.
(f) Optimum pH:
   pH 7.5.
(g) pH stability:
   The enzyme is stable at pH 5.0 to 9.0.
(h) Thermal stability:
   The first β -agarase has thermal stability up to 40 °C.

The second β -agarase of the present invention is an enzyme having the catalytic activity for the hydrolysis of agarose into lower molecular substances, mainly neoagarobiose (disaccharide), and it is identified that the enzyme has the following physico-chemical properties (a) to (g).

(a) Molecular weight:
   The molecular weight was determined by SDS-polyacrylamide gel electrophoresis (SDS-PAGE), with the result that the molecular weight is about 98,000. No report has been issued yet of any known agarase with such molecular weight.
(b) Isoelectric point:
   The result of the isoelectric focusing indicates that the enzyme has an isoelectric point of about 3.4. No report has been issued yet of any known agarase with such isoelectric point.
(c) Solubility:
   The enzyme is water soluble.
(d) Action:
   The enzyme catalyses the hydrolysis of at least agar, agarose and porphyran into low molecular substances, mainly disaccharides. The enzyme does not hydrolyze κ-carrageenan or λ -carrageenan.
(e) Optimum temperature:
   55 °C.
(f) Optimum pH:
   pH 6.5.
(g) pH stability:
   pH 4.0 to 10.0.
(h) Thermal stability:
   The second β -agarase has also thermal stability up to 40 °C.

Either one of the first or second β -agarase according to the invention can be generated by using a microorganism; more specifically, by culturing a microorganism having the potency of generating the first and second β -agarase, the first or second β -agarase can be isolated from the inside of the cultured microorganism or the culture supernatant. As the microorganism for generating either one of the enzymes, use may be made of the aforementioned Vibrio sp. PO-303 newly isolated from marine sediment. The microorganism Vibrio sp. PO-303 can generate the two enzymes, namely the first and second β-agarases, simultaneously. The microorganism to be used in accordance with the invention is not limited to the bacterial strain Vibrio sp. PO-303, or the variant thereof or the modified strain thereof, but any of the microorganisms having the potency of generating the first β -agarase or the second β -agarase may be used as well.

For preparing the first or second β -agarase, a microorganism having the potency of generating the first or second β -agarase is cultured using agar as the carbon source, to isolate and purify the first or second β -agarase from the culture supernatant. As the culture medium, use may be made of any natural medium or synthetic medium containing agar as the carbon source and also containing appropriate amounts of nutrients such as nitrogen source and inorganic compounds and the like.

As the carbon source containing agar, commercially available agar powder should be used; additionally, use may be made singly or in combination of agarose, agaropectin, agar processed substances such as partially hydrolyzed agar products, and red seaweed such as Gelidium spp. and Gracilaria spp. containing unprocessed agarose. In addition to agar, use may be made of sugars such as glucose as the carbon source.

As the nitrogen source, alternatively, use may be made of those employed for routine culture compositions. For example, use may be made of hydrolysates of a variety of animals and plants such as meat extract, peptone, yeast extract, dry yeast, casamino acid, various amino acids, and urea, and an organic or inorganic nitrogen source such as various inorganic ammonium salts and nitrate salts, singly or in combination of two or more thereof.

Furthermore, as such inorganic compound, use may be made of one or more of essential inorganic salts for the microorganism to be cultured, such as the phosphate, hydrochloride, sulfate, carbonate, acetate and the like of metals such as sodium, magnesium, calcium, iron, and manganese.

For the culturing, the culturing method and conditions may be selected, depending on the properties of a microorganism to generate the objective first or second β -agarase. For example, shaking culture, agitation culture in aeration and the like are suitable for the mass culture of any aerobic microorganism.

The first or second β -agarase generated by culturing such microorganism can be isolated and purified by routine methods. When such enzyme is contained in the bacterium, for example, the first or second β -agarase is extracted and purified from the bacterium disrupted with routine means. The first or second β -agarase generated into the culture broth is recovered by purifying the culture supernatant after the removal of the microorganism by filtration, centrifuging and the like.

For such purification, purification methods routinely employed may be applicable. For example, the first or second β -agarase can be precipitated by salting out and solvent precipitation, and then, the resulting precipitate is separated from the solvent to purify the agarase. By subjecting the precipitate to ion exchange chromatography, gel chromatography, isoelectric focusing and the like, the agarase may be purified further.

The first or second β -agarase acts on at least one substrate of agar, agarose or porphyran to generate an oligosaccharide. As has been described above, the oligosaccharide generated from agarose is useful as an investigative reagent; additionally, the oligosaccharide has pharmacological actions such as hypotensive action, anti-tumor action and the action of activating enterokinetis, as well as a bacteriostatic action of a certain microorganism.

When the recovered first or second β -agarase exerts actions on seaweed, the protoplast of the seaweed cell is readily be generated.

Because the first or second β -agarase is an enzyme of hydrolyzing the agarose in the agar composition, the agarase is applicable to the extraction of a desired DNA fragment from agarose gel. For ex ample, a gene in an objective region, which is preliminarily identified by agarose electrophoresis, can be readily extracted by solubilizing the agar around the region with the first or second β -agarase.

### BRIEF DESCRIPTION OF THE DRAWING

Fig. 1 schematically shows the known structure of the units composing agarose.

### DESCRIPTION OF THE PREFERRED EMBODIMENTS

### Example 1 = Harvesting of Vibrio spp. from marine sediment =

A microorganism with a higher β -agarase activity was screened and isolated from microorganisms living in marine sediment. More specifically, by isolating marine microorganisms in marine sediment, culturing the individual microorganisms in culture media containing agar as the carbon source, and assaying the β -agarase activity thereof, a microorganism with the highest β -agarase activity was selected.

The β -agarase activity was assayed as follows. Adding the culture supernatant (0.5 mL) of each of the microorganisms into a 100 mM MES buffer solution, pH 7.5 (0.5 mL) containing 0.5 wt % agar, and then incubating the mixture at 38 °C for 10 minutes, the increase of the reducing sugars in generation was assayed by the Somogyi-Nelson method. The enzyme activity of one unit was defined as the amount of an enzyme exhibiting the reductive potency corresponding to that of D-galactose of 1 µ mol, per minute under the aforementioned reaction conditions.

### Example 2 = Bacteriological performance =

Based on the Bergey's Manual of Systematic Bacteriology, the microorganism of the highest β -agarase activity screened in Example 1, was examined and identified as a marine microorganism of the genus Vibrio. Table 1 shows the morphology of the microorganism, and Table 2 shows the results of the physiological tests.

**Table 1**

| Morphology | |
|---|---|
| Size : | 0.6 to 1.0 × 1.0 to 2.4 µm |
| Shape : | rod |
| Gram stain : | negative |
| Motility : | positive |
| Flagellum : | single pole flagellum |
| Spore formation : | no |
| Atmospheric growth : | positive. |

**Table 2**

| Physiological tests | |
|---|---|
| O-F test : | fermentation |
| Catalase test : | positive |
| Oxidase test : | positive |
| Generation of acids from glucose : | yes |
| Gas generation from glucose : | no |
| Esquirine test : | positive |
| V-P test : | positive |
| Reductive potency of nitrate salts : | yes |
| ONPG degradation potency : | yes |
| Growth in TCBS : | yellow |
| 0/129 sensitivity test : | sensitive |
| Arginine dehydrase : | positive |

It is confirmed from Tables 1 and 2 that the present microorganism is gram negative, having single pole flagellum and being capable of fermentation at O-F test, being positive at catalase and oxidase tests, generating acids with no gas generation in glucose media, and being sensitive to a vibriostatic agent (0/129). Thus, it is indicated that it is highly possible that the present organism is a novel marine bacterium of Vibrio sp.. Therefore, the present bacterium is designated Vibrio sp. PO-303.

As to the Vibrio sp. PO-303, furthermore, examination was made of the growing temperature, the enzyme generation, the NaCl concentration for the growth thereof, and the potency of utilizing sugars and amino acids as carbon sources. The results are shown in Tables 3, 4, 5 and 6. In Tables 3, 4 and 5, the symbols "+" and "-" represent "positive" and "negative", respectively.

**Table 3**

| Temp. | |
|---|---|
| 4 °C : | - |
| 20 °C : | + |
| 30 °C : | + |
| 40 °C : | + |
| 43 °C : | - |
| 45 °C : | - |
| 50 °C : | - |

**Table 4**

| | |
|---|---|
| Amylase : | + |
| Gelatinase : | - |
| Arginase : | - |
| Chitinase : | - |
| G +C content : | 44 mol %. |

**Table 5**

| NaCl concentration for growth (wt %) | |
|---|---|
| 0 : | + |
| 3 : | + |
| 6 : | + |
| 8 : | + |
| 10 : | - |

**Table 6**

| Potency of utilizing sugars and amino acids as carbon sources | |
|---|---|
| Such potency positive : | D-xylose |
| : | D-glucose |
| : | D-arabinose |
| : | D-mannose |
| : | D-galactose |
| : | cellobiose |
| : | D-sorbitol |
| : | lactose |
| : | L-arginine |
| Such potency negative : | sucrose |
| : | D-mannitol |
| : | L-ornithine |
| : | ethanol |
| : | L-α-alanine |
| : | L-histidine |
| : | L-proline |

It is indicated from Tables 3 to 6 that Vibrio sp. PO-303 grows at 20 to 40 °C and that the enzyme generation is as follows: positive gener ation of amylase, negative generation of gelatinase, and arginase and chitinase. It is also confirmed that the G-C content of the DNA is 44 %. Additionally, as to the NaCl concentration (wt %) for the growth, positive growth was confirmed at 0 to 8 wt % NaCl concentrations. It was confirmed that the carbon sources to be utilized with the bacterium were D-xylose, D-glucose, D-arabinose, D-mannose, D-galactose, cellobiose, D-sorbitol, lactose, and L-arginine.

### Example 3 = Culturing =

Vibrio sp. PO-303 requires agar as the substance for inducing the enzyme generation. Therefore, the bacterium was cultured under shaking at 25 °C for 4 days, using a culture medium of a composition as follows: 0.3 wt % agar, 0.3 wt % polypeptone, 0.1 wt % yeast extract, 3 wt % sodium chloride, 0.5 wt % magnesium chloride, 0.2 wt % potassium hydrogen diphosphate, and 0.04 wt % potassium dihydrogen phosphate. Agarase of 66 units could be recovered from the culture medium of 1000 mL.

### Example 4 = Purification of β -agarase =

The β -agarase generated was purified as follows. More specifically, Vibrio sp. PO-303 was cultured at a mass scale under the conditions described above. The resulting culture supernatant was centrifuged. To the culture supernatant was added solid ammonium sulfate at 75 wt % (at the saturation level), and the resulting mixture was left to stand at 4 °C for one day. Then, the resulting precipitate in the culture supernatant was centrifuged. The precipitate was dissolved in a smaller volume of 50 mM MES buffer, pH 7.5, and was then dialyzed against a buffer having the same properties. Subsequently, the solution inside the dialysis bag was used as a salting-out enzyme solution.

The salting-out enzyme solution was fractionated by anion exchange chromatography (Product name; DEAE-Toyopearl 65M, manufactured by Tosoh Co. Ltd., Japan), gel filtration chromatography (Product name; Toyopearl 55, manufactured by Tosoh Co. Ltd., Japan), hydrophobic chromatography (Product name; Ether-Toyopearl 650S, manufactured by Tosoh Co. Ltd., Japan), and anion exchange chromatography (Product name; Mono Q HR 5/5, manufactured by Pharmacia AB, Sweden). Consequently, two types of enzyme fractions, namely the first β -agarase and the second β -agarase, were recovered, on the basis of the difference in molecular weight and isoelectric point.

### Example 5 = Enzyme properties of β -agarase =

The molecular weights, isoelectric points, solubility, optimum pHs, pH stability, and optimum temperatures of the first β -agarase and second β -agarase were determined. The results are shown in the following Table 7. Among the two types of the β -agarases, that is, the first β -agarase (molecular weight of 90,000) was designated "β -agarase A"; and the second β -agarase (molecular weight of 98,000) was designated "β -agarase B".

**Table 7**

| Properties | β -agarase A | β -agarase B |
|---|---|---|
| Molecular weight | 90,000 | 98,000 |
| Isoelectric point | 6.6 | 3.4 |
| Solubility | water-soluble | water-soluble |
| Optimum pH | pH 7.5 | pH 6.5 |
| pH stability | pH 5.0 - 9.0 | pH 4.0 - 10.0 |
| Optimum temperature | 45 °C | 55 °C |

These two enzyme fractions were individually homogeneous on disk electrophoresis. Thus, it is confirmed that these fractions were purified at high purities. Examination was made of the action mechanisms of the purified β -agarase A and β -agarase B with agar, agarose, porphyran, neoagarobiose, neoagarotetraose, and neoagarohexaose. The results are shown in Table 8.

**Table 8**

| Subjects | β -agarase A | β -agarase B |
|---|---|---|
| Agar | 4S > 6S >> 8S > 2S | 2S >> 4S |
| Agarose | 4S > 6S >> 8S > 2S | 2S >> 4S |
| Porphyran | 4S > 6S >> 2S | 2S >> 4S |
| κ -Carrageenan | no hydrolysis | no hydrolysis |
| λ -Carrageenan | no hydrolysis | no hydrolysis |
| Neoagarobiose | no hydrolysis | no hydrolysis |
| Neoagarotetraose | no hydrolysis | 2S |
| Neoagarohexaose | 4S and 2S | 2S |
| Note: 2S denotes "disaccharide", 4S "tetrasaccharide", 6S "hexasaccharide" and 8S "octasaccharide". The disaccharide, tetrasaccharide and hexasaccharide in the table represent neoagarobiose, neoagarotetraose and neoagarohexaose, respectively. The unequal mark is arranged so as to represent the relation of the relative levels of the generated oligosaccharides. | | |

As shown in Tables 7 and 8, it is confirmed that Vibrio sp. PO-303 generates two types of agarases having different action mechanisms, namely the enzymes β -agarase A and β -agarase B; the former β -agarase hydrolyzes agar, agarose, and porphyran to generate mainly a hydrolyzed product of neoagarotetraose (tetrasaccharide) or neoagarohexaose (hexasaccharide), while the latter β -agarase generates a main purified product of neoagarobiose (disaccharide).

### Example 6 = Preparation of neoagarosaccharide =

### 6.1. Preparation of oligosaccharide from agar:

Conventionally, acid hydrolysis has been employed as a method for preparing oligosaccharides from agar. However, the acid hydrolysis requires heating at 100 °C; additionally, the generated oligosaccharides are thereby hydrolyzed simply into monosaccharides over a long duration of the reaction. Therefore, it has been difficult to prepare oligosaccharides efficiently. On the contrary, it is possible to prepare oligosaccharides at a high efficiency at room temperature by the method using the β -agarase A or β -agarase B. It has been confirmed in the following manner that the use of neoagarase hydrolyzing neoagarotetraose, in particular, is advantageous for the preparation of neoagarobiose which has been difficult conventionally.

Firstly, β -agarase B (2 units) prepared by the method of Example 4 was mixed, in a test tube, with agarose (20 mL) adjusted to a concentration of 1 %, for reaction at 38 °C for 20 hours. Subsequently, the reaction products were fractionated by gel filtration on a column (10× 90 cm, Product name; Sephadex G-15, manufactured by Pharmacia AB, Sweden). Consequently, neoagarobiose (about 28 %) was recovered.

### 6.2. Preparation of oligosaccharide from porphyran:

Porphyran is a polysaccharide containing sulfate groups in the chemical skeleton similar to that of agar. Thus, due to the fact that the conventional acid hydrolysis decomposes the sulfate groups in addition to the aforementioned problem, the conventional acid hydrolysis method is also problematic in that the preparation of sulfate group-containing oligosaccharides is difficult. Alternatively, the β -agarase A or β -agarase B prepared in Example 4 as described above conveniently hydrolyzes porphyran with no decomposition of the sulfate groups, to readily generate a variety of oligosaccharides containing sulfate groups.

More specifically, β -agarase B (2 units) prepared by the method of Example 4 was mixed with porphyran (20 mL) adjusted to a concentration of 1 % in a test tube, for reaction at 38 °C for 20 hours. Subsequently, the reaction products were fractionated by gel filtration on a column (10× 90 cm, Product name; Sephadex G-15, manufactured by Pharmacia AB, Sweden). Consequently, neoagarobiose (about 16 %) was recovered.

### Example 7 = Preparation of protoplast from red seaweed =

For the development of a superior industrial species of seaweed, biotechnology by cell fusion and genetic manipulation is excellent technology. So as to carry out the procedures of biotechnology, it is essential the development of a method for preparing protoplasts by solubilizing cell walls. By using the β -agarase A or β -agarase B recovered in Example 4, a very large number of protoplasts can be prepared from a certain species of Rhodophyceae including agar and porphyran as described below.

More specifically, a subject red seaweed (1 g) was charged in a 100-mL beaker containing 5 wt % papaine and a 20 mM MES buffer solution, pH 7.5 (30 mL) containing 0.7 M mannitol, and was then stirred at 22 °C for 30 minutes. Then, the resulting mixture was filtered and washed through a 40-µ m nylon mesh using a MES buffer solution, pH 7.5 containing 0.7 M mannitol, and the resulting washed thallus was cut into pieces of several millimeters with a knife.

The cut pieces (200 mg) were charged in a 30-mL Erlenmeyer flask containing β -agarase A (2 unites) as well as commercially available cellulase Onozuka RS (4 %; manufactured by Kabushiki Kaisha Yakult Honsha, Japan), and Macerzyme R-10 (1 %; manufactured by Kabushiki Kaisha Yakult Honsha, Japan) and a 20 mM MES buffer solution, pH 6.0 containing 0.7 M mannitol. The resulting mixture was then stirred at 22 °C for 5 hours. Consequently, protoplasts were recovered at the following individual numbers: 105 to 106 protoplasts from Grateloupia turuturu, and 104 to 105 protoplasts from Gracilaria verrucosa. It was confirmed that protoplasts could be recovered by means of β -agarase B in the same manner.

### Example 8 = Extraction of DNA fragment from agarose gel =

Even after purification at a high purity, the β -agarase A can liquefy agarose. Thus, the β -agarase A can be used for recovering DNA fragments fractionated by agarose gel electrophoresis. More specifically, the agarose gel after electrophoresis is solubilized with addition of purified β -agarase A, to extract an objective DNA fragment. Because agarose can be liquefied by β -agarase B in the same manner as described above, it is needless to say that β -agarase B is also applicable to the extraction of an objective DNA fragment by solubilizing the agarose gel after electrophoresis.

## Claims

1. A novel β -agarase, having an enzyme activity for catalyzing the hydrolysis of agarose into low molecular substances, mainly neoagarotetraose (tetrasaccharide) and neoagarohexaose (hexasaccharide) and having the following physico-chemical properties (a) to (g):
(a) a molecular weight of about 90,000
(b) an isoelectric point of about 6.6
(c) solubility: water soluble
(d) actions: catalyzing the hydrolysis of at least agarose and porphyran into low molecular substances, mainly tetrasaccharides and hexasaccharides but never hydrolyzing κ -carrageenan or λ -carrageenan
(e) optimum temperature of 45 °C
(f) optimum pH of p H 7.5
(g) pH stability: stable at pH 5.0 to 9.0.

2. A β -agarase according to claim 1, generated by culturing a microorganism having the potency of generating β -agarase, using at least one of carbon source selected from a group consisting of agar, agarose and porphyran.

3. A β -agarase according to claim 1, generated by culturing Vibrio sp. PO-303 (FERM BP-5411) as a novel microorganism.

4. A novel β -agarase, having an enzyme activity for catalyzing the hydrolysis of agarose into low molecular substances, mainly neoagarobiose (disaccharide) and having the following physico-chemical properties (a) to (g)
(a) a molecular weight of about 98,000
(b) an isoelectric point of about 3.4
(c) solubility: water soluble
(d) actions: catalyzing the hydrolysis of at least agarose and porphyran into low molecular substances, mainly disaccharides but never hydrolyzing K -carrageenan or λ -carrageenan
(e) optimum temperature of 55 °C
(f) optimum pH of p H 6.5
(g) pH stability: stable at pH 4.0 to 10.0.

5. A β -agarase according to claim 4, generated by culturing a microorganism having the potency of generating β -agarase, using at least one of carbon source selected from a group consisting of agar, agarose and porphyran.

6. A β -agarase according to claim 4, generated by culturing Vibrio sp. PO-303 (FERM BP-5411) as a novel microorganism.

7. Vibrio sp. PO-303 (FERM BP-5411) as a novel microorganism having the potency of generating β -agarase, wherein the β -agarase has a catalytic activity for the hydrolysis of agarose into low molecular substances, mainly neoagarotetraose (tetrasaccharide) and neoagarohexaose (hexasaccharide) and having the following physico-chemical properties (a) to (g):
(a) a molecular weight of about 90,000
(b) an isoelectric point of about 6.6
(c) solubility: water soluble
(d) actions: catalyzing the hydrolysis of at least agarose and porphyran into low molecular substances, mainly tetrasaccharides and hexasaccharides but never hydrolyzing κ -carrageenan or λ -carrageenan
(e) optimum temperature of 45 °C
(f) optimum pH of p H 7.5
(g) pH stability: stable at pH 5.0 to 9.0.

8. Vibrio sp. PO-303 (FERM BP-5411) as a novel microorganism having the potency of generating β -agarase, wherein the β -agarase has a catalytic activity for the hydrolysis of agarose into low molecular substances, mainly neoagarobiose (disaccharide) and has the following physico-chemical properties (a) to (g):
(a) a molecular weight of about 98,000
(b) an isoelectric point of about 3.4
(c) solubility: water soluble
(d) actions: catalyzing the hydrolysis of at least agarose and porphyran into low molecular substances, mainly disaccharides but never hydrolyzing κ -carrageenan or λ -carrageenan
(e) optimum temperature of 55 °C
(f) optimum pH of p H 6.5
(g) pH stability: stable at pH 4.0 to 10.0.

9. A method for preparing β -agarase, comprising the steps of:
culturing a microorganism having the potency of generating β -agarase using at least one of carbon source selected from a group consisting of agar, agarose and porphyran to obtain culture supernatant containing β -agarase;
isolating said β -agarase from the culture supernatant from said culturing step; and
purifying said β -agarase resulting from said isolating step,
wherein said β -agarase has a catalytic activity for the hydrolysis of agarose into low molecular substances, mainly neoagarotetraose (tetrasaccharide) and neoagarohexaose (hexasaccharide) and has the following physico-chemical properties (a) to (g):
(a) a molecular weight of about 90,000
(b) an isoelectric point of about 6.6
(c) solubility: water soluble
(d) actions: catalyzing the hydrolysis of at least agarose and porphyran into low molecular substances, mainly tetrasaccharides and hexasaccharides but never hydrolyzing κ -carrageenan or λ -carrageenan
(e) optimum temperature of 55 °C
(f) optimum pH of p H 6.5
(g) pH stability: stable at pH 4.0 to 10.0.

10. A method according to claim 9, wherein the microorganism is Vibrio sp. PO-303 (FERM BP-5411).

11. A method for preparing β -agarase, comprising the steps of:
culturing a microorganism having the potency of generating β -agarase using at least one of carbon source selected from a group consisting of agar, agarose and porphyran to obtain culture supernatant containing β -agarase;
isolating said β -agarase from the culture supernatant from said culturing step; and
purifying said β -agarase resulting from said isolating step,
wherein said β -agarase has a catalytic activity for the hydrolysis of agarose into low molecular substances, mainly neoagarobiose (disaccharide) and has the following physico-chemical properties (a) to (g):
(a) a molecular weight of about 98,000
(b) an isoelectric point of about 3.4
(c) solubility: water soluble
(d) actions: catalyzing the hydrolysis of at least agarose and porphyran into low molecular substances, mainly disaccharides but never hydrolyzing κ -carrageenan or λ -carrageenan
(e) optimum temperature of 55 °C
(f) optimum pH of p H 6.5
(g) pH stability: stable at pH 4.0 to 10.0.

12. A method according to claim 11, wherein the microorganism is Vibrio sp. PO-303 (FERM BP-5411).

13. A method for preparing an oligosaccharide, comprising reacting β -agarase with at least one substrate of agar, agarose or porphyran, wherein the β -agarase has a catalytic activity for the hydrolysis of agarose into low molecular substances, mainly neoagarotetraose (tetrasaccharide) and neoagarohexaose (hexasaccharide) and has the following physico-chemical properties (a) to (g):
(a) a molecular weight of about 90,000
(b) an isoelectric point of about 6.6
(c) solubility: water soluble
(d) actions: catalyzing the hydrolysis of at least agarose and porphyran into low molecular substances, mainly tetrasaccharides and hexasaccharides but never hydrolyzing κ -carrageenan or λ -Carrageenan
(e) optimum temperature of 55 °C
(f) optimum pH of p H 6.5
(g) pH stability: stable at pH 4.0 to 10.0.

14. A method for preparing an oligosaccharide, comprising reacting β -agarase with at least one substrate of agar, agarose or porphyran, wherein the β -agarase has a catalytic activity for the hydrolysis of agarose into low molecular substances, mainly neoagarobiose (disaccharide) and has the following physico-chemical properties (a) to (g):
(a) a molecular weight of about 98000
(b) an isoelectric point of about 3.4
(c) solubility: water soluble
(d) actions: catalyzing the hydrolysis of at least agarose and porphyran into low molecular substances, mainly disaccharides but never hydrolyzing κ -carrageenan or λ -carrageenan
(e) optimum temperature of 55 °C
(f) optimum pH of p H 6.5
(g) pH stability: stable at pH 4.0 to 10.0.

15. A method for preparing the protoplast of seaweed cells, comprising stirring seaweed cells together with β -agarase in a buffer solution, wherein said β -agarase has a catalytic activity for the hydrolysis of agarose into lower molecular substances, mainly neoagar otetraose (tetrasaccharide) and neoagarohexaose (hexasaccharide) and has the following physico-chemical properties (a) to (g):
(a) a molecular weight of about 90,000
(b) an isoelectric point of about 6.6
(c) solubility: water soluble
(d) actions: catalyzing the hydrolysis of at least agarose and po rphyran into low molecular substances, mainly tetrasaccharides and hexasaccharides but never hydrolyzing κ -carrageenan or λ -carrageenan
(e) optimum temperature of 45 °C
(f) optimum pH of p H 7.5
(g) pH stability: stable at pH 5.0 to 9.0.

16. A method for preparing the protoplast of seaweed cells, comprising stirring seaweed cells together with β -agarase in a buffer solution, wherein said β -agarase has a catalytic activity for the hydrolysis of agarose into low molecular substances, mainly neoagarobiose (disaccharide) and has the following physico-chemical propert ies (a) to (g):
(a) a molecular weight of about 98000
(b) an isoelectric point of about 3.4
(c) solubility: water soluble
(d) actions: catalyzing the hydrolysis of at least agarose andpo rphyran into low molecular substances, mainly disaccharides but never hydrolyzing κ -carrageenan or λ -carrageenan
(e) optimum temperature of 55 °C
(f) optimum pH of p H 6.5
(g) pH stability: stable at pH 4.0 to 10.0.
